# EUROPEAN PATENT SPECIFICATION

(11) **EP 1 073 427 B1**
(45) Date of publication and mention of the grant of the patent: **17.11.2004**
(21) Application number: 99919391.5
(22) Date of filing: 28.04.1999
(51) Int. Cl.: A61K 9/70, A61K 38/44, A61K 38/19

(54) **PERIADVENTITIAL DELIVERY DEVICE**
PERIADVENTITIALES VERABREICHUNGSSYSTEM
DISPOSITIF D'ADMINISTRATION PERIADVENTITIELLE

(30) Priority: 28.04.1998 GB 9809082; 14.05.1998 GB 9810429
(43) Date of publication of application: 07.02.2001
(73) Proprietor: Ark Therapeutics Limited, London W1T 6DE (GB)
(72) Inventor: MARTIN, John Francis, The Cruciform Project, London W1P 9LN (GB); YLÄ-HERTTUALA, Seppo, FIN-70211 Kuopio (FI); BARKER, Stephen G. E., The Middlesex Hospital, London W1N 8AA (GB)
(74) Representative: Perry, Robert Edward
(86) International application number: PCT/GB1999/001310
(87) International publication number: WO 1999/055315

(56) References cited:
- WO-A-96/38188
- WO-A-98/20027
- US-A- 3 797 485
- SONG C X ET AL: "Controlled release of U-86983 from double-layer biodegradable matrices: effect of additives on release mechanism and kinetics" JOURNAL OF CONTROLLED RELEASE, vol. 45, no. 2, 17 March 1997 (1997-03-17), page 177-192 XP004099366 ISSN: 0168-3659
- VILLA A E ET AL: "LOCAL DELIVERY OF DEXAMETHASONE FOR PREVENTION OF NEOINTIMAL PROLIFERATION IN A RAT MODEL OF BALLOON ANGIOPLASTY" JOURNAL OF CLINICAL INVESTIGATION, vol. 93, no. 3, 1 March 1994 (1994-03-01), pages 1243-1249, XP002043082 ISSN: 0021-9738

## Description

### Field of the Invention

The present invention relates to a device that can be used for delivering an active agent, in therapy.

### Background of the Invention

Intimal hyperplasia is the increase in the number of cells between the endothelium and internal elastic lamina of a blood vessel, particularly in the intimal layer found there, or in an artery. Intimal hyperplasia is often caused by smooth muscle cell (SMC) proliferation in the blood vessel wall.

When intimal hyperplasia occurs, *de novo* thickening of the intimal layer or ofthe vessel wall, i.e. stenosis, may result. Thus, the blood vessel may become occluded.

Also, when an obstruction in a blood vessel has been cleared, intimal hyperplasia occurring after surgery may lead to the artery's becoming occluded again. This is known as restenosis.

Intimal hyperplasia, whether it leads to stenosis or restenosis, remains a major problem after various surgical procedures.

GB-A-2298577 discloses a non-restrictive, porous, external stent for arteriovenous bypass grafting procedures. This stent has beneficial effects on luminal size and on medial and intimal thickening.

WO-A-9423668 discloses a device for the local delivery of an agent into a blood vessel, including a reservoir formed between two elements thereof. Its use requires implantation, i.e. cutting through the vessel and then securing the device to the vessel walls. The device is partially porous. The reservoir is in direct contact with luminal blood flow. This involves the risk of infection.

US-A-3797485 discloses a device for delivering a drug to the adventitial surface of a blood vessel. It is provided with permanent walls and transcutaneous tubes for the delivery of drug in liquid form. The intention is that the drug should pass to another site.

US-A-5 540928 and related patent publications (inventors: Edelman *et al*) disclose an extraluminal device in the form of a disc comprising a polymer matrix, with a central hole. In order to ensure that the agent, e.g. heparin, is delivered at the blood vessel wall, a radial hole may be bored in the coating; see Edelman *et al*, PNAS USA 87:3773-7 (May 1990).

WO-A-9820027 describes experiments in which a collar was placed around the outside of the artery of a rabbit. This procedure normally causes intimal hyperplasia in the rabbit artery, leading to thickening of the arterial wall, which is similar to the stenosis that can occur in human arteries following bypass operations. When the collar was used to deliver DNA encoding VEGF to the arterial wall using a plasmid/liposome vector, the VEGF gene was overexpressed in the arterial wall, including the endothelial layer. Intimal hyperplasia was inhibited. It has been found that adventitial delivery is suitable for all tested systems.

These findings demonstrate that effective agents can be delivered to the exterior of the blood vessel, to treat intimal hyperplasia. This has several advantages. In particular, the therapeutic agent is not washed away from the site of the hyperplasia by blood flow as with intralumenal delivery. A delivery reservoir can be maintained around the blood vessel, and there is no need for any intralumenal manipulations which damage the endothelium of the blood vessel (and can themselves trigger intimal hyperplasia).

### Summary of the Invention

According to the present invention, a product comprises a biodegradable matrix material, a nucleic acid and a glue, as defined in claim 1 or claim 4.

### Description of the Invention

Various agents, including peptidic and non-peptidic compounds, genes that can express active products, agents that stimulate NO or prostacyclin production, agonists of a receptor to which VEGF binds, etc, are suitable for use in the invention. As described in WO-A-9820027, an illustrative agent is the VEGF protein or nucleic acid. Herein, references to such agents, and to VEGF itself, are given by way of example.

Nucleic acids may be delivered in a "naked" form unassociated with a vector, of by means of a gene therapy vector. It is preferred to deliver them by means of any suitable gene therapy vector. In particular, viral or non-viral vectors may be used.

The body part to which the invention may be applied is typically a duct, and will typically be essentially tubular or cylindrical. For example, it may be a nerve, Fallopian tube, bile duct, aortic aneurism or blood vessel. In particular, anti-thrombotic agents may be administered to act on blood platelets or the coagulation cascade, growth factors to the nerves, and anti-rejection agents to transplanted organs.

The active agent is delivered to the outside of the body part to be treated, e.g. artery.

In use of this invention, treatment preferably comprises surgical exposure of the body part; application, around the part, of a strip of a matrix material including, or to include, the agent; covering the matrix material with a glue; and closure of the surgical wound.

Particularly in this latter aspect, the agent may be contained within a medium within the device, e.g. a solid or gel medium. This may help to prevent the agent escaping into the tissue.

For example, a sheet or strip of a biodegradable material may be impregnated with the therapeutic agent. The strip is cut to a desired size, before or after being wound around the body part to be treated. It is then sealed *in situ* by the application of, for example, a tissue glue around the matrix material. The glue may advantageously be activated remotely, e.g. by light.

As indicated above, the therapeutic agent that is used in the present invention may be a nucleic acid from which a gene product is derived, *in situ*, e.g. following transport across the wall of the body part to which the device is applied. By way of example, a suitable gene may be provided in a polymer solution. If it is desired that a long-acting effect is provided, continuous expression of a gene may be provided, e.g. using fibroblasts.

The present invention may be understood with reference to the accompanying drawing, in which:

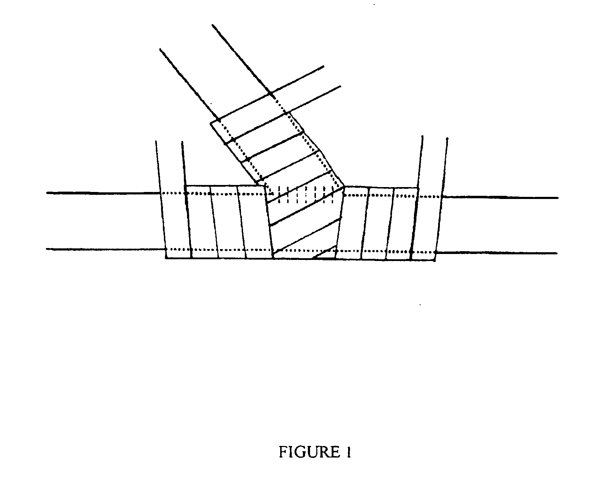

Figure 1 is a schematic view of a "wrap" embodying the invention in place around an arterial anastomosis.

Particularly where a strip of flexible matrix material is used as a wrap, it may be provided in a kit with a glue and the agent. These components may be separate, or two or more may be combined. Thus, the agent may be pre-impregnated in the matrix material. The material may be in bi-layer form, one layer being of the matrix and the other of a relatively impermeable material, e.g. both of collagen but of different characteristics. Any or each component may be aseptically packaged, in generally known manner, ready for use.

The sealant may be a conventional "tissue glue", such as the thrombin glue sold under the name Tisseal, or a cyanomethacrylate-based glue.

The matrix material is, advantageously, biodegradable over a set time course, for example a period of 1 to 5 days, by which time the active agents in the formulation are likely to have become exhausted. The material is also chosen so as not to promote too severe a reaction from the surrounding tissue. Examples of suitable materials for the body include gelatin, alginate or collagen. These materials also allow the body flexibility and enable the device to be manufactured by molding or extrusion.

The outer layer may, for example, be made of solid collagen and the inner layer made of sponge-like collagen cross-linked thereto, the sponge-like layer being capable of being impregnated with the pharmaceutical formulation containing the agent to be delivered. In such a situation, the device may be provided to the surgeon for fitment with the formulation already impregnated therein, or it may be wetted with the formulation after fitment, for example by being injected as described earlier.

As indicated above, VEGF proteins or nucleic acids may be used for the treatment or prevention of intimal hyperplasia arising from any clinical circumstances. For example, it is possible to treat hyperplasia arising after any type of surgical procedure, including angioplasty, for example balloon angioplasty; bypass surgery, such as coronary bypass surgery in which a vein is anastomosed to an artery; other anastomosis procedures, for example anastomosis in the legs; and endarteriectomy, for example carotid artery endarteriectomy. It is also possible to treat intimal hyperplasia associated with arterial damage or hypertension, for example pulmonary artery hypertension. The invention provides for treatment of intimal hyperplasia in any type of blood vessel, e.g. in an artery or vein, preferably an artery.

According to the invention, it is possible to treat or ameliorate established intimal hyperplasia or to prevent intimal hyperplasia from arising. Similarly, it is possible to diminish the likelihood of intimal hyperplasia arising, or to diminish the severity of established intimal hyperlasia or hyperplasia that is likely to arise. Treatment according to the invention may take place before, during, or after a surgical procedure, for example in order to reduce the chance of hyperplasia arising after the procedure.

Preferably, the VEGF nucleic acid or protein is administered with a view to preventing or treating *de novo* stenosis. It can, however, also be used to treat or prevent restenosis.

The proteins or nucleic acids of the invention are preferably delivered in the form of a pharmaceutical formulation comprising a pharmaceutically acceptable carrier. Any suitable pharmaceutical formulation may be used.

For example, suitable formulations may include aqueous and non-aqueous sterile injection solutions which may contain anti-oxidants, buffers, bacteriostats, bactericidal antibiotics and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a frozen or freeze-dried (lyophilized) condition requiring only the addition of the sterile liquid carrier, for example water for injection, immediately prior to use.

It should be understood that, in addition to the ingredients particularly mentioned above, formulations of this invention may include other agents conventional in the art having regard to the type of formulation in question. Of the possible formulations, sterile pyrogen-free aqueous and non-aqueous solutions are preferred.

The proteins, nucleic acids and vectors may be delivered in any suitable dosage, and using any suitable dosage regime. Those of skill in the art will appreciate that the dosage amount and regime may be adapted to ensure optimal treatment of the particular condition to be treated, depending on numerous factors. Some such factors may be the age, sex and clinical condition of the subject to be treated.

For the delivery of naked nucleic acids encoding VEGF or constructs comprising such nucleic acids, typical doses are from 0.1-5000 µg, for example 50-2000 µg, such as 50-100 µg, 100-500 µg or 500-2000 µg per dose. For the delivery of VEGF protein, suitable doses include doses of from 1 to 1000 µg for example from 1 to 10 µg, from 10 to 100 µg, from 100 to 500 µg or from 500 to 1000 µg.

One embodiment of the invention involves the perivascular delivery of liposomally-associated human VEGF₁₆₅ gene to the popliteal artery of patients with severe peripheral vascular disease undergoing above-knee amputation. This may comprise placing a perivascular gene delivery system in the form of a wrap in position around the popliteal artery and sealing with tissue glue.

The agents to be administered, e.g. an aqueous solution of gene polasmid/liposome complexes, is delivered locally to the target tissue by soaking a strip of collagen sheet with the solution immediately before it is applied to the popliteal artery.

The collagen wrap is a strip cut from surgical collagen sheet of 25 mm long and 4-5 mm wide. It is saturated with 2.0 ml of solution of the agent, containing the dose of gene plasmid, and then wrapped around a 25 mm long segment of the popliteal artery. It is then covered completely with two layers of surgical sealant.

## Claims

1. A product comprising:
a biodegradable matrix material in a form that can be wrapped around a body part;
a nucleic acid capable of expressing a therapeutic agent, to treat a condition *via* delivery to the adventitial surface of a body part, the nucleic acid being in a form that can be taken up by the matrix material; and
a glue;
for combined use in the treatment of the condition, by using the glue to form a seal around the matrix material when impregnated with the nucleic acid.

2. A product according to claim 1, wherein the matrix material is impregnated with the nucleic acid.

3. A product according to claim 1, wherein the matrix material and the nucleic acid are separate.

4. A product comprising, aseptically packaged, a biodegradable matrix material as defined in claim 1, impregnated with a nucleic acid as defined in claim 1, and a glue.

5. A product according to any preceding claim, wherein the form of the matrix material is a flexible strip.

6. A product according to any preceding claim, wherein the matrix material comprises collagen.

7. A product according to any preceding claim, wherein the body part is a nerve or blood vessel.

8. A product according to any preceding claim, wherein the agent stimulates NO or prostacyclin production

9. A product according to claim 8, for the treatment or prevention of stenosis induced by a surgical procedure or associated with pulmonary artery hypertension

10. A product according to claim 9, wherein the surgical procedure is angioplasty, coronary bypass surgery, surgical anastomosis or endarterectomy.

11. A product according to claim 7, for the treatment or prevention of stenosis or restenosis of the blood vessel

12. A product according to any of claims 8 to 11, wherein the agent is an agonist of a receptor to which VEGF binds.

13. A product according to claim 8, for the treatment of hypertension, e.g. essential hypertension, primary pulmonary hypertension or cor pulmonale

14. A product according to any preceding claim, wherein the nucleic acid is in association with a viral or non-viral vector.

## Patentansprüche

1. Produkt, umfassend:
ein biologisch abbaubares Matrixmaterial in einer Form, welche um einen Teil eines Körpers herumgewickelt werden kann;
eine Nukleinsäure, welche in der Lage ist, ein therapeutisches Mittel zu exprimieren, um einen Zustand oder ein Leiden über eine Abgabe an die adventitielle Oberfläche eines Teils eines Körpers zu behandeln, wobei die Nukleinsäure in einer Form vorliegt, die durch das Matrixmaterial aufgenommen werden kann; und
einen Klebstoff;
für eine kombinierte Verwendung bei der Behandlung des Zustands oder Leidens, indem der Klebstoff verwendet wird, um eine Abdichtung oder Versiegelung um das Matrixmaterial herum, wenn dieses mit der Nukleinsäure imprägniert ist, zu bilden.

2. Produkt nach Anspruch 1, wobei das Matrixmaterial mit der Nukleinsäure imprägniert ist.

3. Produkt nach Anspruch 1, wobei das Matrixmaterial und die Nukleinsäure getrennt vorliegen.

4. Produkt, umfassend, aseptisch verpackt, ein biologisch abbaubares Matrixmaterial, wie in Anspruch 1 definiert, welches mit einer Nukleinsäure, wie in Anspruch 1 definiert, imprägniert ist, und einen Klebstoff.

5. Produkt nach einem der vorangegangenen Ansprüche, wobei die Form des Matrixmaterials ein flexibler Streifen ist.

6. Produkt nach einem der vorangegangenen Ansprüche, wobei das Matrixmaterial Collagen umfasst.

7. Produkt nach einem der vorangegangenen Ansprüche, wobei der Teil eines Körpers ein Nerv oder Blutgefäß ist.

8. Produkt nach einem der vorangegangenen Ansprüche, wobei das Mittel die NO- oder Prostacyclinproduktion stimuliert.

9. Produkt nach Anspruch 8 für die Behandlung oder Verhütung einer Stenose, welche durch einen operativen Eingriff induziert wird oder mit Pulmonalarterien-Hypertonie verbunden ist.

10. Produkt nach Anspruch 9, wobei der operative Eingriff eine Angioplastie, eine aorto-koronare Bypass-Operation, eine chirurgische Anastomose oder eine Endarteriektomie ist.

11. Produkt nach Anspruch 7 für die Behandlung oder Verhütung einer Stenose oder Restenose des Blutgefäßes.

12. Produkt nach einem der Ansprüche 8 bis 11, wobei das Mittel ein Agonist eines Rezeptors, an den VEGF bindet, ist.

13. Produkt nach Anspruch 8 für die Behandlung von Hypertonie, z.B. essentieller Hypertonie, primärer pulmonaler Hypertonie oder Cor pulmonale.

14. Produkt nach einem der vorangegangenen Ansprüche, wobei die Nukleinsäure in Kombination mit einem viralen oder nicht-viralen Vektor vorliegt.

## Revendications

1. Produit comprenant :
une matière de matrice biodégradable sous une forme qui peut être enroulée autour d'une partie du corps ;
un acide nucléique capable d'exprimer un agent thérapeutique pour traiter un état par apport à la surface adventitielle d'une partie du corps, l'acide nucléique étant sous une forme qui peut être absorbée par la matière de matrice ; et
une colle ;
pour un usage conjoint dans le traitement de l'état, en utilisant la colle pour former un joint autour de la matière de matrice lorsqu'elle est imprégnée avec l'acide nucléique.

2. Produit selon la revendication 1, dans lequel la matière de matrice est imprégnée avec l'acide nucléique.

3. Produit selon la revendication 1, dans lequel la matière de matrice et l'acide nucléique sont séparés.

4. Produit comprenant une matière de matrice biodégradable telle que définie dans la revendication 1, imprégnée avec un acide nucléique tel que défini dans la revendication 1, et une colle, emballés aseptiquement.

5. Produit selon l'une quelconque des revendications précédentes, dans lequel la forme de la matière de matrice est un ruban souple.

6. Produit selon l'une quelconque des revendications précédentes, dans lequel la matière de matrice comprend du collagène.

7. Produit selon l'une quelconque des revendications précédentes, dans lequel la partie du corps est un nerf ou un vaisseau sanguin.

8. Produit selon l'une quelconque des revendications précédentes, dans lequel l'agent stimule la production de NO ou de prostacycline.

9. Produit selon la revendication 8, pour le traitement ou la prévention de sténose provoquée par une opération chirurgicale ou associée à une hypertension des artères pulmonaires.

10. Produit selon la revendication 9, dans lequel l'opération chirurgicale est une angioplastie, un pontage coronarien, une anastomose chirurgicale ou une endartériectomie.

11. Produit selon la revendication 7, pour le traitement ou la prévention de sténose ou de resténose du vaisseau sanguin.

12. Produit selon l'une quelconque des revendications 8 à 11, dans lequel l'agent est un agoniste d'un récepteur auquel se lie VEGF.

13. Produit selon la revendication 8, pour le traitement de l'hypertension, par exemple de l'hypertension artérielle essentielle, de l'hypertension artérielle pulmonaire primitive ou du coeur pulmonaire.

14. Produit selon l'une quelconque des revendications précédentes, dans lequel l'acide nucléique est associé à un vecteur viral ou non viral.
